# EUROPÄISCHE PATENTSCHRIFT

(11) **EP 1 176 963 B1**
(45) Veröffentlichungstag und Bekanntmachung des Hinweises auf die Patenterteilung: **15.12.2004**
(21) Anmeldenummer: 00927101.6
(22) Anmeldetag: 28.04.2000
(51) Int. Cl.: A61K 31/472, A61K 31/495, A61K 31/496, A61K 31/506, A61K 31/5513, A61P 13/12

(54) **VERWENDUNG VON DOPAMIN-D 3-REZEPTORANTAGONISTEN ZUR HERSTELLUNG VON ARZNEIMITTEL FÜR DIE BEHANDLUNG VON NIERENFUNKTIONSSTÖRUNGEN**
USE OF DOPAMINE D 3 RECEPTOR ANTAGONISTS FOR PRODUCING MEDICAMENTS FOR TREATING KIDNEY DISORDERS
UTILISATION DE ANTAGONISTES DU RECEPTEUR DE LA DOPAMINE D 3 POUR LA PRODUCTION DE MEDICAMENTS SERVANT AU TRAITEMENT DE DYSFONCTIONNEMENTS RENAUX

(30) Priorität: 07.05.1999 DE 19922443
(43) Veröffentlichungstag der Anmeldung: 06.02.2002
(73) Patentinhaber: Abbott GmbH & Co. KG, 65205 Wiesbaden (DE)
(72) Erfinder: MÜHLBAUER, Bernd, D-72108 Rottenburg (DE); GROSS, Gerhard, D-67346 Speyer (DE); STARCK, Dorothea, D-67059 Ludwigshafen (DE); TREIBER, Hans-Jörg, D-68782 Brühl (DE)
(74) Vertreter: Kinzebach, Werner, Dr.
(86) Internationale Anmeldenummer: PCT/EP2000/003865
(87) Internationale Veröffentlichungsnummer: WO 2000/067847

(56) Entgegenhaltungen:
- WO-A-97/25324
- US-A- 3 270 004
- LUIPPOLD GERD ET AL: "Dopamine D3 receptor activation modulates renal function in anesthetized rats." NAUNYN-SCHMIEDEBERG'S ARCHIVES OF PHARMACOLOGY, Bd. 358, Nr. 6, Dezember 1998 (1998-12), Seiten 690-693, XP000986638 ISSN: 0028-1298
- LUIPPOLD G ET AL: "Increase of renal vascular resistance by dopamine D3-receptor activation in anesthetized rats." NAUNYN-SCHMIEDEBERG'S ARCHIVES OF PHARMACOLOGY, Bd. 357, Nr. 4 SUPPL, 1998, Seite R116 XP000986707 39th Spring Meeting of the German Society for Experimental and Clinical Pharmacology and Toxicology;Mainz, Germany; March 17-19, 1998 ISSN: 0028-1298
- BARILI P ET AL: "Microanatomical localization of the dopamine D3 receptor subtype in the rat kidney." CLINICAL AND EXPERIMENTAL PHARMACOLOGY AND PHYSIOLOGY, Bd. 26, Nr. SUPPL., April 1999 (1999-04), Seite S67 XP000986705 Seventh International Conference on Peripheral Dopamine;Dublin, Ireland; August 3-5, 1998 ISSN: 0305-1870
- LUIPPOLD G ET AL: "Renal effects of dopamine D3 receptor activation in spontaneously hypertension rats (SHR)." KIDNEY & BLOOD PRESSURE RESEARCH, Bd. 21, Nr. 2-4, 1998, Seite 114 XP000986708 Congress of Nephrology 1998 Joint Scientific Meeting of the Society Nephrology;Erlangen, Germany; September 19-22, 1998 ISSN: 1420-4096
- HUSSAIN TAHIR ET AL: "Renal dopamine receptor function in hypertension." HYPERTENSION (DALLAS), Bd. 32, Nr. 2, August 1998 (1998-08), Seiten 187-197, XP000986719 ISSN: 0194-911X
- ASICO LAUREANO D ET AL: "Disruption of the dopamine D3 receptor gene produces renin-dependent hypertension." JOURNAL OF CLINICAL INVESTIGATION, Bd. 102, Nr. 3, 1. August 1998 (1998-08-01), Seiten 493-498, XP000986718 ISSN: 0021-9738
- MURRAY, P. JOHN ET AL: "A Novel series of arylpiperazines with high affinity and selectivity for the dopamine D3 receptor" BIOORG. MED. CHEM. LETT. (1995), 5(3), 219-22 , XP004135762 in der Anmeldung erwähnt

## Beschreibung

Die vorliegende Erfindung betrifft die Verwendung von selektiven Dopamin-D₃-Rezeptorliganden zur Herstellung von Arzneimitteln zur Behandlung von Nierenfunktionsstörungen.
Die Rezeptorliganden sind Rezeptor-Antagonisten.
Insbesondere betrifft die Erfindung Arzneimittel zur Behandlung von Nierenfunktionsstöruhgen, bei denen eine Störung der glomerulären Filtrationsrate im Sinne einer glomerulären Hyperfiltration auftritt.
Dopamin spielt bei der Regulation der renalen Natrium-Ausscheidung eine wichtige Rolle. Es konnte gezeigt werden, daß Dopamin-Rezeptoren (D_{1A}, D_{1B} als D₁-artige, D₂, und D₃ als D₂artiger Rezeptor-Subtyp) in Nieren vorkommen (T. Hussein und M.F. Lokwandala, Hypertension, Bd. 32, 1998, Seiten 187-197).

So gilt es als erwiesen, daß der Dopamin-D₃-Rezeptor in der Niere, insbesondere im Nephron, exprimiert ist (vgl. D.P. O'Connell et al., Hypertension, 1998, 32, 886). In P. Barili et al., Clinical and Experimental Pharmacology and Physiology, Bd. 26, Suppl. 1999, Seite S67 wird berichtet, daß [³H]-7-OH-DPAT, ein Dopamin-D₃-Rezeptor-Agonist, spezifisch an bestimmte Orte in Rattennieren band.

In der DE-A 4223921 ist ganz allgemein die Verwendung von Dopamin-Rezeptor-Ancagonisten ohne Hinweis auf bestimmte Subtypen in der Therapie der fortschreitenden Verschlechterung der Nierenfunktion beschrieben.

G. Luippold et al. berichten in Naunyn-Schmiedeberg's Archives of Pharmacology, Bd. 357, Nr. 4 Suppl. 1998, Seite R116, daß die Aktivierung des Dopamin-D₃-Rezeptors mit R(+)-7-Hydroxydipropylaminotetralin (7-OH-DPAT) in anästhesierten Ratten zu einer Erhöhung des renalen Gefäßwiderstands und der glomerulären Filtrationsrate führt.

G. Luippold et al. beschreiben des Weiteren in Naunyn-Schmiedeberg's Arch. Pharmacol (1998) 358:690-693 Untersuchungen hinsichtlich des Einflusses bestimmter D₂- und D₃- Rezeptor-Antagonisten auf die renale Hämodynamik und Ausscheidungsfunktion in einem rein artifiziellen Funktionszustand ohne pathophysiologische Veränderungen.

**L.D. Asico et al.,** J. Clin. **Invest.,** Vol. 102 **(1998), 493-498, beschreiben, daß eine Blockade des D**_{**3**}**-Rezeptors eine erhöhte Renin-Produktion, renale Natrium-Retention und dadurch eine** reninabhängige Hypertonie zur Folge hat. G. Luippold et al. berichten in Kidney & Blood Pressure Research, Bd. 21, 1998, Seite 114, daß die Verabreichung von 7-OH-DPAT an SHR- und Wistar-Kyoto-Ratten Natriurese und Diurese sowie einen Anstieg der glomerulären Filtrationsrate verursachte.

In WO 97/25324 beispielsweise sind Dopamin-D₃-Rezeptor-Liganden zur Behandlung von Schizophrenie und Parkinson'scher Erkrankung vorgeschlagen. P.J. Murray, Bioorg. Med. Chem. Lett., Bd. 513), 1995, Seiten 219-222, beschreiben Arylpiperazine mit Selektivität für den Dopamin-D₃-Rezeptor zur Behandlung psychiatrischer Erkrankungen.

Sklerosierende Prozesse in den glomerulären Kapillaren und dadurch verursachte Störungen der Filtrationsrate treten bei Erkrankungen wie Diabetes mellitus, Hypertonie, infektiösen oder nichtinfektiösen Glomerulonephritiden, aufsteigenden Harnwegsinfekten, Sichelzellanämie, oder kompensatorischer Hypertrophie nach unilateraler Nierenresektion auf.

Die fortschreitende Verschlechterung der Nierenfunktion betrifft nahezu alle Patienten mit Glomerulonephritis und mehr als ein Drittel aller Patienten mit Diabetes mellitus.

Nierenfunktionsstörungen treten aufgrund einer auch als diabetische Nephropathie bezeichneten Glomerulosklerose auf, welche histologisch durch eine diffuse Verdickung der glomerulären Kapillaren und eine Veränderung des Mesangiums durch diffuse Einlagerung von basalmembranartigem Material oder kugelförmigen fibrinösen Einlagerungen gekennzeichnet ist. Als Folge kommt es zu einer Veränderung der Filtrationswirkung im Sinne einer Hyperfiltration.

Auch bei Glomerulonephritiden kommt es zu einer Veränderung der glomerulären Basalmembranen und als Folge zu einer Störung der Filtrationsrate.

Aufgabe der vorliegenden Erfindung war es, Arzneimittel zu finden, die eine gezielte Therapie der Nierenfunktionsstörungen bei den genannten Erkrankungen ermöglichen.

Demgemäß wurde die eingangs definierte Verwendung gefunden. So betrifft die vorliegende Erfindung die Verwendung von Dopamin-D₃-Rezeptorliganden zur Herstellung von Arzneimitteln zur Behandlung von Nierenfunktionsstörungen, wobei der Rezeptorligand ein Rezeptor-Antagonist ist.

Zu erfindungsgemäß behandelbaren Nierenfunktionsstörungen gehören Nierenfunktionsstörungen mit glomerulärer Hyperfiltration; durch Diabetes mellitus hervorgerufene Nierenfunktionsstörungen; durch nichtrenale Hypertonie verursachte Nierenfunktionsstörungen; durch Glomerulonephritis verursachte Nierenfunktionsstörungen; durch aufsteigende Harnwegsinfekte hervorgerufene Nierenfunktionsstörungen; durch Sichelzellanämie hervorgerufene Nierenfunktionsstörungen, durch kompensatorische Hypertrophie nach unilateraler Nephrektomie hervorgerufene Nierenfunktionsstörungen; durch mesangiale Dysfunktion hervorgerufene Nierenfunktionsstörungen.

Insbesondere betrifft die vorliegende Erfindung die Verwendung von Dopamin-D₃-Rezeptor-Antagonisten zur Herstellung von Arzneimitteln zur Behandlung von diabetischer Nephropathie.

Als Dopamin-D3-Rezeptorliganden eignen sich grundsätzlich alle Verbindungen mit einer Affinität zu diesem Rezeptor, bevorzugt solche Verbindungen, die eine um einen Faktor 10 größere Affinität zu diesem Rezeptor im Vergleich zu einem anderen Dopamin-Rezeptor aufweisen.

Geeignete Verbindungen sind beispielsweise die in den folgenden Dokumenten genannten selektiven Dopamin-D3-Rezeptorliganden: 2-Aminoindane wie in der WO 95/04713 beschrieben, Benzimidazole wie aus der WO 95/30658 bekannt, 2-Aminotetraline wie aus der EP-A 286516 und Bioorg. Med. Chem. Lett. 1997, 7, 881 bekannt, ebenso wie die aus WO 94/21608, WO 96/30333, Bioorg. Med. Chem. Lett. 1996, 6, 6403, oder J. Med. Chem.,1996, 39, 4233, bekannten Verbindungen.

Weiterhin eignen sich Tetrahydroisochinolin-Derivate. wie sie in den Dokumenten WO97/43262, WO 98/51671, WO 98/50363, WO 98/49145, WO 98/50364 oder WO 98/06699 beschrieben sind, sowie die aus der WO 97/17326 oder der WO 97/47602 bekannten Verbindungen.

Ebenso eignen sich die in den Dokumenten WO 97/34884, Bioorg. Med. Chem. Lett. 1997, 7, 2403, EP-A 779584, WO 98,/18798, oder WO 96/02520, WO 96/02519, WO 96/02249, WO 96/02246, WO 97/00106 und WO 98/04138 genannten Verbindungen.

Verbindungen der allgemeinen Formel I

L - D - E (I)

worin
- L: für
einen 5- oder 6-gliedrigen aromatischen Heteromonocyclus L1 mit 1, 2 oder 3 Heteroatomen, die unabhängig voneinander ausgewählt sind unter O, N und S,
oder einen aromatischen oder heteroaromatischen Ring ausgewählt unter der Gruppe L2 steht 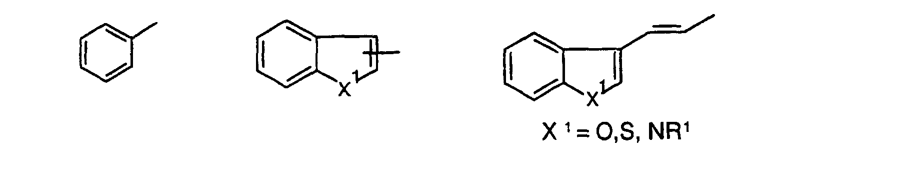 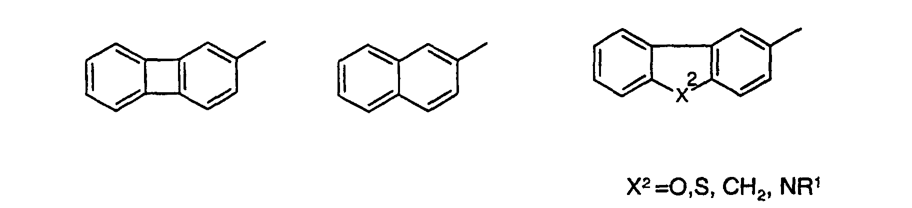 wobei L gegebenenfalls 1, 2, 3 oder 4 Substituenten aufweist, die unabhängig voneinander ausgewählt sind unter OR₁, C₁-C₆-Alkyl, das gegebenenfalls durch OH, OC₁-C₆-Alkyl, Phenyl oder Halogen substituiert ist wie CF₃, CHF₂, C₂-C₆-Alkenyl, C₂-C₆-Alkinyl, C₃-C₈-Cycloalkyl, Halogen, CN, CONR¹R², CO₂R¹, NO₂, NR¹R², SR¹, SO₂R¹, SO₂NR¹R², OSO₂R¹, Ax1 oder Phenoxy, das gegebenenfalls durch C₁-C₆-Alkyl, OC₁-C₆-Alkyl, oder Halogen substituiert ist, C₁-C₆-Alkanoyl oder Benzoyl;
worin
- Ax1: für Phenyl, Naphthyl oder einen 5- oder 6-gliedrigen heterocyclischen aromatischen Ring mit 1 bis 4 Heteroatomen, die ausgewählt sind unter O, S und N, steht, wobei Ax1 gegebenenfalls 1, 2, 3 oder 4 Substituenten aufweist, die unabhängig voneinander ausgewählt sind unter C₁-C₆-Alkyl, das gegebenenfalls durch OH, OC₁-C₆-Alkyl, Halogen oder Phenyl substituiert ist, C₁-C₆-Alkoxy, C₂-C₆-Alkenyl, C₂-C₆-Alkinyl, C₃-C₆-Cycloalkyl, Halogen, CN, COOR¹, NR¹R², NO₂, SR¹, SO₂R², SO₂NR¹R², oder Phenyl, das gegebenenfalls durch C₁-C₆-Alkyl, OC₁-C₆-Alkyl, NR²R², CN, CF₃, CHF₂, oder Halogen substituiert ist, und wobei der erwähnte heterocyclische, aromatische Ring gegebenenfalls mit einem Phenylring kondensiert sein kann, steht;
R¹ für H, C₃-C₆-Cycloalkyl, oder C₁-C₆-Alkyl, das gegebenenfalls durch OH, OC₁-C₆-Alkyl, Phenyl oder Halogen, beispielsweise als CF₃ oder CHF₂ substituiert ist, steht;
die Reste R², die gleich oder verschieden sein können, die für R¹ angegebenen Bedeutungen besitzen oder für COR¹ oder CO₂R¹ stehen;
R³ für Ax1, OR¹, R¹, C₂-C₆ Alkenyl, C₂-C₆-Alkinyl, Hal, CN, CONR¹R¹, COOR¹, NO₂, NR¹R¹, SR¹, OSO₂R¹, SO₂R¹, steht,
R⁴ bis R⁶ unabhängig voneinander für H, C₁-C₆-Alkyl, OR¹, CN, NR²R², SR¹, CF₃, stehen;
R⁷ für H, C₁-C₆-Alkyl, C₃-C₆-Cycloalkyl oder Phenyl steht;
- D: für CONR⁷-C₃-C₁₀-Alkylengruppe steht, wenn L für L2 steht, oder, wenn L einen 5- oder 6-gliedrigen aromatischen Heteromonocyclus L1 bedeutet, für eine C₄-C₁₀-Alkylengruppe oder eine C₃-C₁₀-Alkylengruppe steht, die wenigstens eine Gruppe Z umfaßt, die ausgewählt ist unter -CH₂-, O, S, NR¹, C₃-C₆-Cycloalkyl, CO, CONR¹, einer Doppel- und einer Dreifachbindung, wobei R¹ wie oben definiert ist,
- E: einen der Reste der Formel (E1) oder (E2) bedeutet und
(E1) für B-G
steht,
worin
- B für: einen 6-, 7- oder 8-gliedrigen gesättigten Ring mit einem oder zwei Stickstoffheteroatomen steht, wobei sich die Stickstoffheteroatome in 1,4- oder 1,5-Position befinden und der Ring in 1-Position an den Rest D und in 4- oder 5-Position an den Rest G gebunden ist und wobei der Ring außerdem eine Doppelbindung in 3- oder 4-Position aufweisen kann; 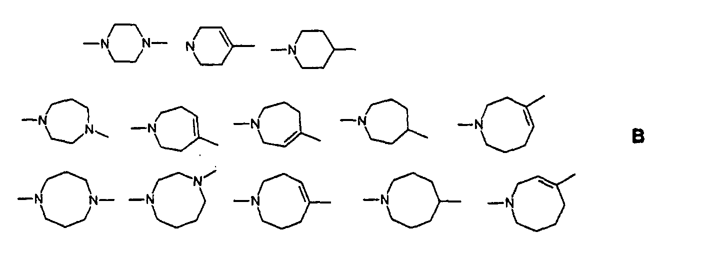
worin
- G für: Phenyl, Pyridyl, Pyrimidinyl oder Triazinyl steht, wobei G gegebenenfalls 1 bis 4 Substituenten aufweisen kann, die unabhängig voneinander ausgewählt sind unter OR¹, Alkyl, C₂-C₆-Alkenyl, C₂-C₆-Alkinyl, Alkoxyalkyl, Halogenalkyl, Halogen, CN, CO₂R¹, NO₂, SO₂R¹, NR¹R², SO₂NR¹R², SR¹, einem 5-oder 6-gliedrigen carbocyclischen, aromatischen oder nicht-aromatischen Ring und einem 5- oder 6-gliedrigen heterocyclischen aromatischen oder nicht-aromatischen Ring mit 1 oder 2 Heteroatomen, die ausgewählt sind unter 0, S und N, wobei der carbocyclische oder der heterocyclische Ring gegebenenfalls substituiert ist durch C₁-C₆-Alkyl, Phenyl, Phenoxy, Halogen, OC₁-C₆-Alkyl, OH, NO₂ oder CF₃, wobei G gegebenenfalls mit einem carbocyclischen oder heterocyclischen Ring der oben definierten Art kondensiert sein kann;
und (E2) für eine der Reste E2a bis E2d steht 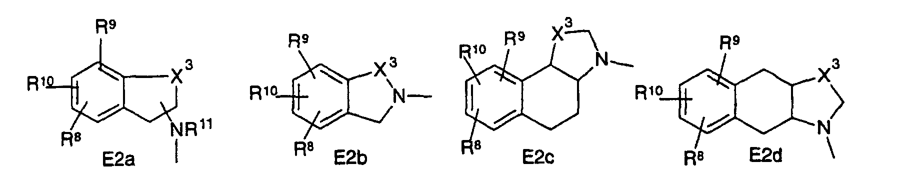 worin X³ für CH₂ oder CH₂CH₂ steht;
R¹¹ für H, C₁-C₆-Alkyl, das gegebenenfalls durch OH, OC₁-C₆-Alkyl, Phenyl oder Halogen substituiert ist, C₃-C₆-Cycloalkyl, gegebenenfalls halogensubstituiertes (1 oder 2 Halogenatome) C₂-C₆-Alkenyl oder für C₂-C₆-Alkinyl steht;
R⁸, R⁹ und R¹⁰ unabhängig voneinander ausgewählt sind unter H, C₂-C₆-Alkyl, das gegebenenfalls durch OH, OC₁-C₆-Alkyl, C₁-C₆-Alkylthio, Halogen oder Phenyl substituiert ist, OH, C₁-C₆-Alkoxy, SH, C₁-C₆-Alkylthio, C₂-C₆-Alkenyl, C₂-C₆-Alkinyl, Halogen, CN, NO₂, SO₂R¹, OSO₂R¹, SO₂NR¹R¹, NHSO₂R¹, NR¹R², einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 oder 2 Heteroatomen, die unabhängig voneinander ausgewählt sind unter O, S und N, wobei der carbocyclische oder der heterocyclische Ring ein oder zwei Substituenten aufweisen kann, die unabhängig voneinander ausgewählt sind unter C₁-C₆-Alkyl, Phenyl, Phenoxy, Halogen, C₁-C₆-Alkoxy, OH, NO₂, CF₃ und CHF₂ und wobei zwei der Substituenten R⁸, R⁹ und R¹⁰ zusammen mit den Kohlenstoffatomen des Phenylringes, an den sie gebunden sind, einen an den Phenylring ankondensierten Phenyl-, Cyclopentyl- oder Cyclohexylring bilden können; sowie deren Salze mit physiologisch verträglichen Säuren.

Im Rahmen der vorliegenden Erfindung besitzen die nachfolgenden Ausdrücke die anschließend angegebenen Bedeutungen:

Alkyl (auch in Resten wie Alkoxy, Alkylamino etc.) bedeutet eine geradkettige oder verzweigte Alkylgruppe mit 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH, OC₁-C₆-Alkyl, Halogen oder Phenyl.

Beispiele für eine Alkylgruppe sind Methyl, Ethyl, n-Propyl, i-Propyl, n-Butyl, iso-Butyl, t-Butyl, etc.

Cycloalkyl steht insbesondere für C₃-C₆-Cycloalkyl, wie Cyclopropyl, Cyclobutyl, Cyclopentyl und Cyclohexyl.

Alkylen steht für geradkettige oder verzweigte Reste. Wenn D keine Gruppe Z aufweist, umfaßt D 4 bis zu 10 Kohlenstoffatome, bevorzugt 4 bis 8 Kohlenstoffatome. Die Kette zwischen L und Gruppe E weist dann mindestens vier Kohlenstoffatome auf. Wenn eine der genannten Gruppen Z aufweist, umfaßt D 3 bis zu 10 Kohlenstoffatome, vorzugsweise 3 bis 8 Kohlenstoffatome.

Die Alkylengruppen können gegebenenfalls wenigstens eine der oben bei der Definition von D angegebenen Gruppen Z umfassen. Diese kann - ebenso wie die erwähnte Doppel- oder Dreifachbindung - in der Alkylenkette an beliebiger Stelle oder in Position 1 oder 2 der Gruppe D (vom Rest L her gesehen) angeordnet sein. Die Reste CONR¹ und COO sind vorzugsweise so angeordnet, daß jeweils die Carbonylgruppe der Gruppe L1 zugewandt ist. Besonders bevorzugt steht D für Verbindungen nach der Formel I, worin D für -Z-C₃-C₆-Alkylen, insbesondere -Z-CH₂CH₂CH₂-, - ZCH₂CH₂CH₂CH₂-, -Z-CH₂CH=CHCH₂-, -Z-CH₂C(CH₃)=CHCH₂-, - Z-CH₂C(=CH₂)CH₂-, -Z-CH₂CH(CH₃)CH₂- oder für einen linearen - Z-C₇-C₁₀-Alkylenrest steht. Z kann dabei auch CH₂ bedeuten und steht vorzugsweise für CH₂, O und insbesondere S.

Halogen bedeutet allgemein F, Cl, Br oder I, vorzugsweise F oder Cl.

Halogenalkyl kann ein oder mehrere, insbesondere 1, 2, 3 oder 4 Halogenatome umfassen, die sich an einem oder mehreren C-Atomen befinden können, vorzugsweise in α- oder ω-Position. Besonders bevorzugt sind CF₃, CHF₂, CF₂Cl oder CH₂F.

Acyl steht vorzugsweise für HCO oder C₁-C₆-Alkyl-CO, insbesondere Acetyl.

Wenn L substituiert ist, kann sich der Substituent auch an dem Stickstoffheteroatom befinden.

Vorzugsweise steht L für eine Gruppe der Formel 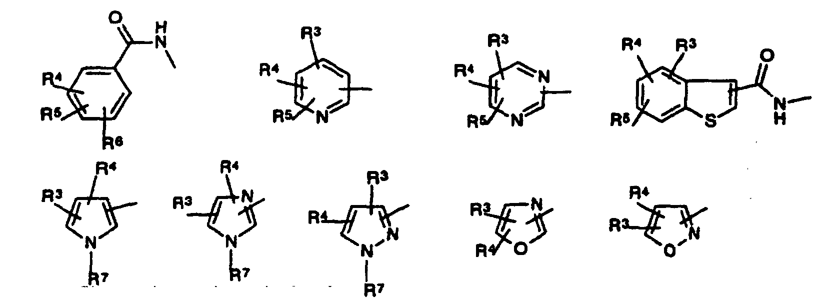 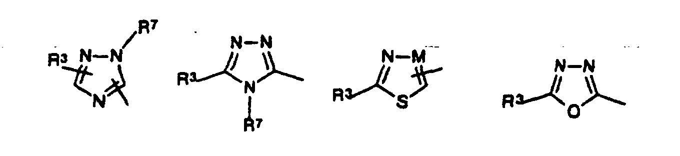 worin
- R³: für Ax1, OR¹, R¹, C₂-C₆ Alkenyl, C₂-C₆-Alkinyl, Hal, CN, CONR¹R¹, COOR¹, NR¹R², SR¹, SO₂R¹ stehen,
- R⁴ bis R⁶: unabhängig voneinander für H, C₁-C6-Alkyl, OR¹, CN, NR²R², SR¹, CF₃ stehen
- R⁷: für H, C₁-C₆-Alkyl oder C₃-C₆-Cycloalkyl steht und
- M: für N oder CH steht.
- Ax1: steht bevorzugt für einen Substituenten der Formel 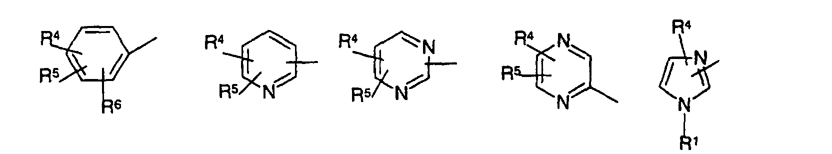 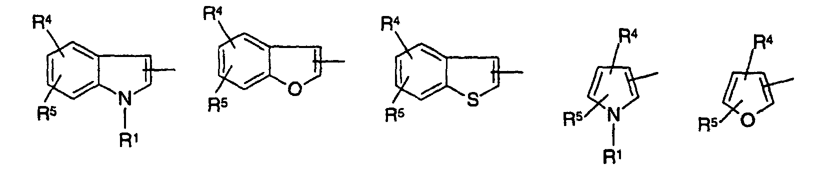 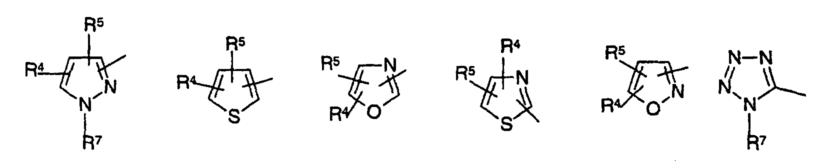 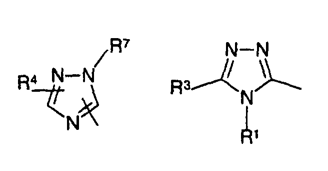
worin
- R⁴ bis R⁶: die oben angegebenen Bedeutungen besitzen und
- R⁷: bevorzugt für C₁-C₄-Alkyl steht.

Besonders bevorzugt steht L für 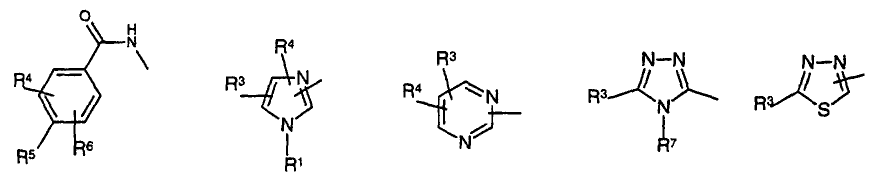 worin
- R³: für Ax1, R¹, COOR¹, NO₂, NR¹R², SR¹, OSO₂CF₃, SO₂R¹, CF₃, CHF₂ steht,
- R⁴: bis R⁶ für H, C₁-C₆-Alkyl, OR¹, NR²R², steht und
- R⁷: für H, C₁-C₆-Alkyl steht.
- Ax1: steht besonders bevorzugt für

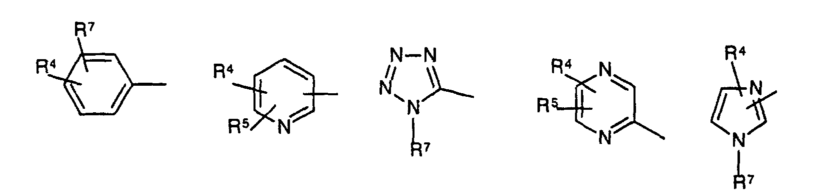

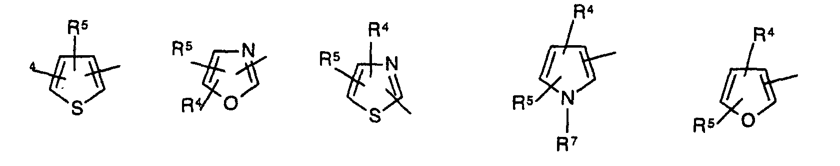

Die angegebenen Phenyl-, Pyrazinyl-, Thiazolyl- und Pyrrolylreste sind insbesondere bevorzugt, worin R⁴, R⁵ und R⁷ die oben angegebenen Bedeutungen besitzen.

Gemäß einer Ausführungsform betrifft die Erfindung die Verwendung von Verbindungen der Formel (I),worin E für die oben genannten Reste (E2)steht und L und D die oben angegebene Bedeutung besitzen.
- L: steht dann vorzugsweise für ein L1a

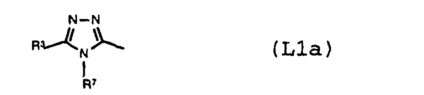

Besonders bevorzugt steht E für E2b worin X³ bevorzugt für CH₂CH₂ steht oder für E2c, worin X³ bevorzugt für CH₂ steht, und L dann besonders bevorzugt für ein 1,2,4-(4H)-Triazol, das einen Substituenten Ax1 in 3-Position und einen Rest R⁷ in 4-Position trägt. Wenn E für eine Gruppe E2b, E2c oder E2d steht, steht D vorzugsweise für C₄-C₁₀-Alkylen oder C₃-C₁₀-Alkylen, das wenigstens eine Gruppe Z umfaßt, die ausgewählt ist unter 0, S, CO, -CH₂-, einer Doppel- und einer Dreifachbindung.

Vorzugsweise steht mindestens einer der Reste R⁸, R⁹ und R¹⁰ für H.

Die Reste R⁸, R⁹ und R¹⁰ sind vorzugsweise und unabhängig voneinander ausgewählt unter H, C₁-C₆-Alkyl, OH, C₁-C₆-Alkoxy, OSO₂R¹, C₁-C₆-Alkylthio-C₁-C₆-alkyl und Halogen. Besonders bevorzugt weist die Phenylgruppe einen oder zwei Substituenten auf, d.h. einer oder zwei der Reste R⁸, R⁹ und R¹⁰ stehen für C₁-C₆-Alkyl, OH, C₁-C₆-Alkoxy oder Halogen.

Wenn einer der Reste R⁸, R⁹ und R¹⁰ für einen 5- oder 6-gliedrigen heterocyclischen Ring steht, so handelt es sich beispielsweise um einen Pyrrolidin-, Piperidin-, Morpholin-, Pyridin-, Pyrimidin-, Triazin-, Pyrrol-, Thiophen- oder Pyrazolrest, wobei ein Pyrrol-, Pyrrolidin-, Pyrazol- oder Thienylrest bevorzugt ist.

Wenn einer der Reste R⁸, R⁹ und R¹⁰ für einen carbocyclischen Rest steht, handelt es sich insbesondere um einen Phenyl-, Cyclopentyl- oder Cyclohexylrest.

Die Gruppe E steht vorzugsweise für eine Gruppe der Formel (E1)
mit (E1) B-G
- B: steht dann vorzugsweise für einen Rest der Formel (B1) 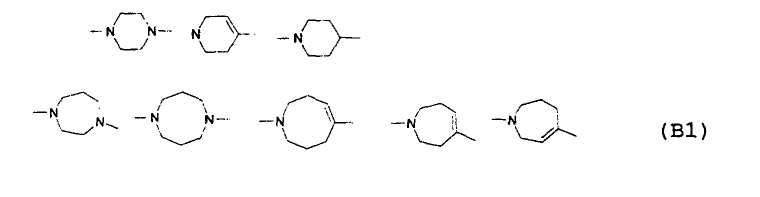 und besonders bevorzugt für (B2)

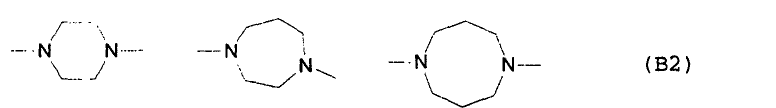

Der Rest G kann einen, zwei, drei oder vier Substituenten, vorzugsweise einen oder zwei Substituenten, die sich insbesondere in m-Stellung und/oder p-Stellung befinden, aufweisen. Vorzugsweise sind sie unabhängig voneinander ausgewählt unter C₁-C₆-Alkyl, Halogenalkyl, NO₂, Halogen, insbesondere Chlor, Phenyl, Pyrrolyl, Imidazolyl, Pyrazolyl, Thienyl, Cyclopentyl und Cyclohexyl. Wenn einer der Substituenten für C₁-C₆-Alkyl steht, ist eine verzweigte Gruppe und insbesondere Isopropyl oder t-Butyl bevorzugt.

Vorzugsweise steht G für gegebenenfalls substituiertes Phenyl, 2-, 3- oder 4-Pyridinyl oder 2-, 4(6)- oder 5- Pyrimidinyl.

Wenn einer der Substituenten des Restes G für einen 5- oder 6-gliedrigen heterocyclischen Ring steht, so handelt es sich beispielsweise um einen Pyrrolidin-, Piperidin-, Morpholin-, Pyridin-, Pyrimidin-, Triazin-, Pyrrol-, Thiophen-, Thiazol-, Imidazol-, Oxazol-, Isoxazol-, Pyrazol-, oder Thiadiazolrest, wobei ein Pyrrol-, Imidazol-, Pyrazol- oder Thienylrest bevorzugt ist.

Wenn einer der Substituenten des Restes G für einen carbocyclischen Rest steht, handelt es sich insbesondere um einen Phenyl-, Cyclopentyl- oder Cyclohexylrest.

Wenn G mit einem carbocyclischen Rest kondensiert ist, handelt es sich insbesondere um einen Naphthalin-, Di- oder Tetrahydronaphthalinrest.

Insbesondere bevorzugt sind Verbindungen der Formel I in denen
- L: ausgewählt ist unter 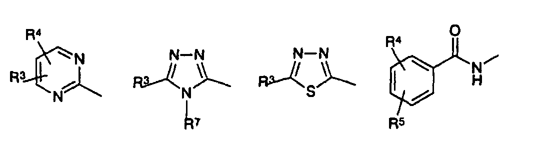 und, D für -Z-(CH₂)₃- oder -Z-(CH₂)₄,und E für B1-G steht, wobei R³, R⁴, R⁷, Z, B1 und G die oben angegebene Bedeutung besitzen.

Ganz besonders bevorzugt sind im Sinne dieser Erfindung Verbindungen, in denen
- L: ausgewählt ist unter 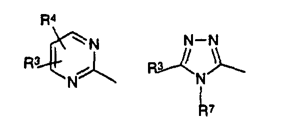
- D: für -Z-(CH₂)₃- oder -Z-(CH₂)₄,
- B: für 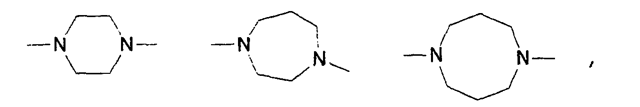 insbesondere 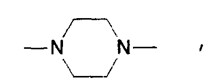 und
- G: für -4-pyrimidyl steht, und die übrigen Reste die oben genannte Bedeutung besitzen,
wobei im Falle, daß L Triazolyl bedeutet, R³ bevorzugt für 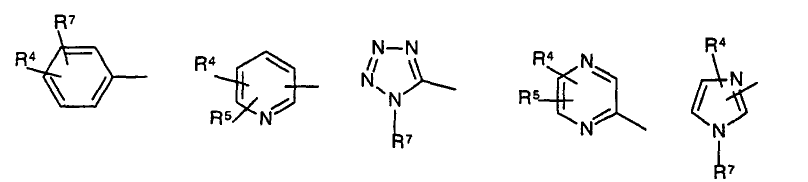 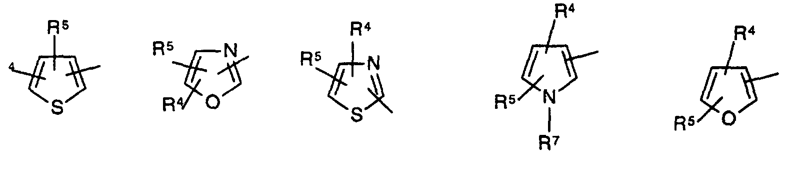 oder NR¹R¹, wobei R¹ gleich oder verschieden sein kann, steht.

Die Erfindung umfaßt auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff, Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Das Verfahren zur Herstellung der Verbindungen der allgemeinen Formel (I) besteht darin, daß man
a) eine Verbindung der allgemeinen Formel (II)

   L - D - Y¹ (II)

   worin Y¹ für eine übliche Abgangsgruppe wie beispielsweise Hal, Alkansulfonyloxy, Arylsulfonyloxy etc. steht, und Z die oben genannten Bedeutungen besitzt, mit einer Verbindung der allgemeinen Formel (III)

   H - E (III)

   umsetzt; oder
b) eine Verbindung der allgemeinen Formel (IV)

   L - D1 - Z¹H (IV)

   worin Z¹ für 0, NR¹ oder S und D1 für C₁-C₁₀-Alkylen oder eine Bindung steht, mit einer Verbindung der allgemeinen Formel V

   Y¹ - D2 - E (V)

   wobei Y¹ die oben angegebene Bedeutung besitzt und D2 für C₂-C₁₀-Alkylen steht, wobei D1 und D2 zusammen 3 bis 10 C-Atome aufweisen,
   umsetzt; oder
c) eine Verbindung der allgemeinen Formel (VI)

   L - Y¹ (VI)

   worin Y¹ die oben angegebene Bedeutung besitzt, mit einer Verbindung der allgemeinen Formel VII

   H - Z¹ - D - E (VII)

   worin Z¹ die oben angegebenen Bedeutungen besitzt, umsetzt; oder
d) eine Verbindung der Formel (VIII)

   NC - D - E (VIII)

   in eine Verbindung des Typs (IX) 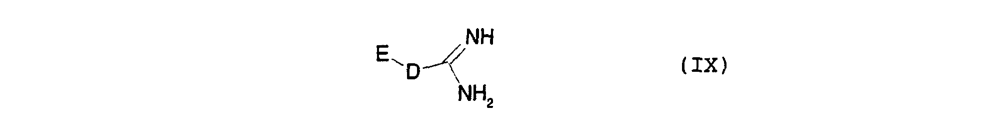 überführt,
   und diese mit einer Dicarbonyl-Verbindung in bekannter Weise umsetzt,
e) daß man eine Verbindung der allgemeinen Formel (X) 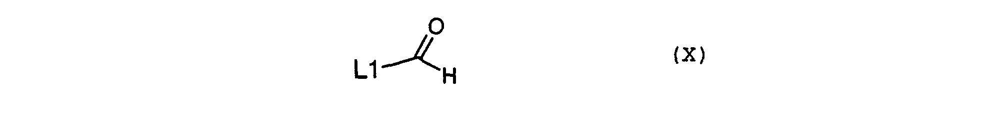 mit literaturbekannten Reagenzien, wie z.B. 1,3-Propandithiol, KCN/Wasser, TMSCN (Trimethylsilylcyanid) oder KCN/Morpholin, wie z.B. beschrieben in
   Albright, *Tetrahedron*, 1983, 39, 3207 oder D. Seebach, *Synthesis* 1969, 17 und 1979, 19 oder H. Stetter, *Angew. Chem. Int*. Ed. 1976, 15, 639 oder van Niel et al., *Tetrahedron* 1989, 45, 7643 Martin et al., *Synthesis* 1979, 633,
   zu den Produkten (Xa) (exemplarisch mit 1,3-Propandithiol) 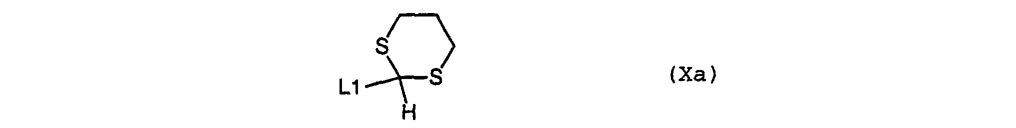 umpolt und anschließend mit Verbindungen der allgemeinen Formel (XI)

   Y¹ - D3 - E (XI)

   wobei Y¹ die oben angegebene Bedeutung besitzt und D3 für C₃-C₉-Alkylen steht, das eine Gruppe Z enthalten kann, kettenverlängert, wobei man nach Entschützen oder Reduktion Verbindungen der Formel (Ia)

   T - Z³ - D2 - E (Ia)

   worin Z³ für CO oder eine Methylengruppe steht und Z³ und D2 zusammen 4 bis 10 C-Atome aufweisen, erhält; oder
g) eine Verbindung der Formel (X) mit einer Verbindung der allgemeinen Formel (XII)

   Y³ - D - E (XII)
worin Y³ für ein Phosphoran oder einen Phosphonsäureester steht, analog zu üblichen Methoden, wie zum Beispiel beschrieben in Houben Weyl *"Methoden der Organischen Chemie"* 4. Auflage, Thieme Verlag Stuttgart, Band V/1b S.383 ff oder Bd V/1c S.575 ff, umsetzt.

Das Verfahren zur Herstellung einer Verbindung der Formel I, die die Gruppe COO oder CONR⁷ enthält umfaßt, besteht darin, daß man eine Verbindung der allgemeinen Formel (XIII) 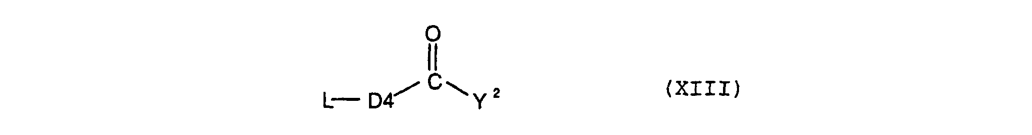 worin Y² für OH, OC₁-C₄-Alkyl, Cl oder zusammen mit CO für eine aktivierte Estergruppe steht, und worin D4 für C₀-C₉-Alkylen, das eine Gruppe Z enthalten kann, steht, mit einer Verbindung der Formel (XIV)

Z² - D - E (XIV)

worin Z² für OH oder NR⁷ steht, umsetzt.

Die Verbindungen der Formel III sind Ausgangsverbindungen zur Herstellung von Verbindungen der Formeln V, VII, VIII.

Verbindungen der Formel IIIa

H - B - G (IIIa)

werden hergestellt durch Standardmethoden, wie z.B. beschrieben in J.A. Kiristy et al., *J. Med. Chem*. 1978, *21,* 1303 oder C.B. Pollard, *J. Am. Chem. Soc.* 1934, *56,* 2199, oder indem man
a) eine Verbindung der allgemeinen Formel (XV)

   HB3 (XV)

   worin B3 für 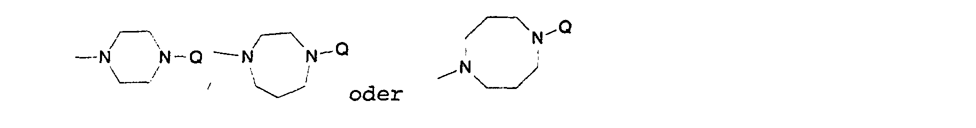 und Q für H oder eine übliche Aminoschutzgruppe z.B. Butyloxycarbonyl, Benzyl oder Methyl, steht, mit
   einer Verbindung der allgemeinen Formel (XVI)

   Y⁴ - G (XVI)

   worin Y⁴ für B(OH)₂, -SnBu₃, Trifluormethansulfonyloxy steht oder die für Y¹ angegebenen Bedeutungen besitzt in bekannter Weise umsetzt; oder
b) eine Verbindung der allgemeinen Formel (XVII)

   Q - B4 (XVII)

   worin B4 für 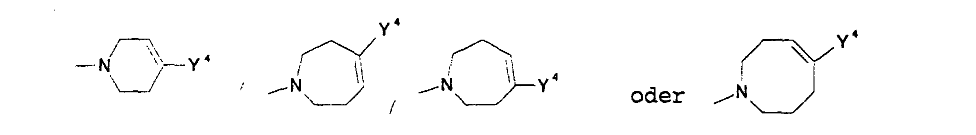 steht und Y⁴ und Q die oben angegebene Bedeutung besitzen
   mit einer Verbindung der allgemeinen Formel (XVIII)

   Y⁵ - G (XVIII)

   worin Y⁵ für Borderivate, wie z.B. B(OH)₂ oder eine metallhaltige Abgangsgruppe, z.B. SnR₃ (R = Butyl oder Phenyl) oder Zinkhalogenid steht, wenn Y⁴ für Halogen oder Trifluormethylsulfonyloxy steht; oder worin Y⁵ für Halogen oder Trifluormethylsulfonyloxy steht, wenn Y⁴ für Borderivate, wie z.B. B(OH)₂ oder eine metallhaltige Abgangsgruppe, z.B. SnR³ (R = Butyl oder Phenyl) oder Zinkhalogenid, steht, nach bekannten Verfahren umsetzt, wie z.B. beschrieben in
   S. Buchwald et al., *Angew. Chem.* 1995, *107*, 1456 oder J.F. Hartweg et al., *Tetrahedron Lett* 1995, *36,* 3604 bzw. J.K. Stille et al., *Angew. Chem*. 1986, *98,* 504 oder Pereyre M. et al., "Tin in Organic Synthesis", Butterworth 1987; oder
c) eine Verbindung der allgemeinen Formel (XIX) 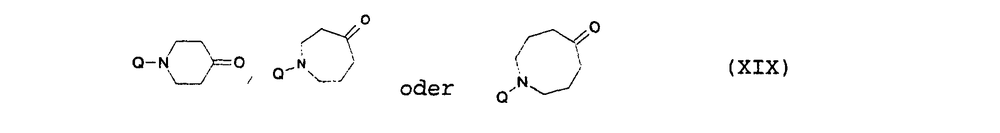 mit einer Verbindung M - G,
worin M für ein Metall wie z.B. Li, MgY⁶ und Y⁶ für Brom, Chlor oder Jod steht, umsetzt.
M-G kann nach literaturbekannten Methoden erhalten werden.

Verbindungen des Typs B sind entweder bekannt oder sie können analog zu bekannten Verfahren hergestellt werden, wie z.B.
1,4- und 1,5-Diazacycloalkane:
L. Börjeson et al. Acta Chem. Scand. 1991, 45, 621 Majahrzah et al Acta Pol. Pharm., 1975, 32, 145
1,4-Diazacyclooct-6-ene:
W. Schroth et al. Z. Chem. 1969, 9, 143 1-Azacyclooctanone:
N.J. Leonard et al. J. Org. Chem. 1964, 34, 1066
1-Azacyclo-heptanone:
A. Yokoo et al. Bull Chem. Soc. Jpn. 1956, 29, 631

Verbindungen des Typs L sowie G sind entweder bekannt oder können nach bekannten Verfahren hergestellt werden wie z.B. beschrieben in A.R. Katritzky, CW. Rees (ed.) "Comprehensive Heterocyclic Chemistry", Pergamon Press, oder "The Chemistry of Heterocyclic Compounds", J. Wiley & Sons Inc. NY und der dort zitierten Literatur oder der vorstehend zitierten Patentliteratur Ein Verfahren zu Herstellung von Verbindungen der allgemeinen Formel (Ib)

L1a - D - E2 (Ib)

besteht darin, daß man
a) eine Verbindung der allgemeinen Formel (XX)

   L1a - D - C(O) - A (XX)

   mit einer Verbindung der Formel E2
   worin A für H oder OH steht,
   unter reduktiven Bedingungen analog literaturbekannten Methoden, wie z.B. beschrieben in *J*. *Org. Chem.* 1986, 50, 1927; oder WO 92/20655 umsetzt, oder
b) zur Herstellung einer Verbindung der Formel (Ib1)

   L1a - D - E2a (Ib1)

   mit Verbindungen der allgemeinen Formel (XXII) bzw. (XXIII), 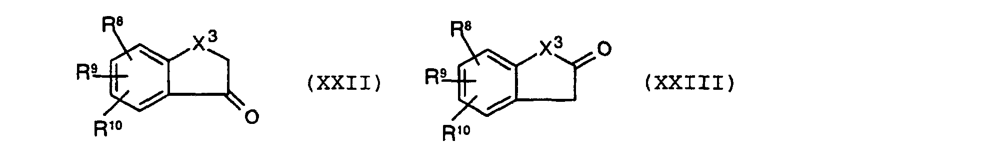 mit einer Verbindung der allgemeinen Formel (XXIV)

   L1a - D - Z⁴H (XXIV)

   wobei Z⁴ für NR¹¹ steht und R¹¹ die oben angegebene Bedeutung besitzt unter reduktiven Bedingungen umsetzt.

Verbindungen des Typs (XXIV) können synthetisiert werden, indem man Verbindungen der Formel (II) mittels Gabriel-Synthese zu dem entsprechenden Amin (XXV)

L - D - NH₂ (XXV)

umsetzt, dann zunächst mit R¹¹ (mit dem entsprechenden Aldehyd oder mittels Alkylierung in Gegenwart einer Base) und anschließend mit E2 in einer reduktiven Aminierung verknüpft, wie z.B. beschrieben in *J. Org. Chem.* 1986, 50, 1927.

Verbindungen der allgemeinen Formel (XXV) können auch erhalten werden durch Umsetzung von Verbindungen der Formel (II) mit Aziden, wie z.B. Natriumazid, und anschließender Reduktion, wie z.B. beschrieben in
H. Staudinger, *Helv. Chim. Acta* 1985, 2, 635 oder
R. Carrie, *Bull. Chem. Soc. Fr.* 1985, 815.

Verbindungen der Formel (XXII) und (XXIII) sind entweder literaturbekannt oder können nach bekannten Methoden hergestellt werden, wie z.B. beschrieben in
A. van Vliet et al. *J. Med*. *Chem*. 1996, 39, 4233
M. Langlois *Bioorg*. *Med*. *Chem. Lett*. 1993, 3, 2035
U. Hacksell *J. Med. Chem*. 1993, 36, 4221
oder in WO 93/08799 oder WO 95/04713.

Wenn E2 für einen Rest der allgemeinen Formel (XXVI) 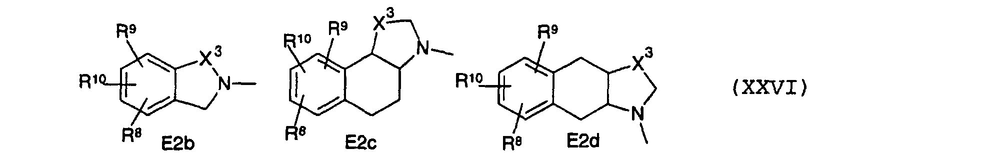 steht, worin R⁸, R⁹, R¹⁰, X³ die oben angegebenen Bedeutungen besitzen, können die entsprechenden Amine hergestellt werden wie z.B. beschrieben in
S. Smith et al., *Bioorg. Med. Chem.* Lett. 1998, 8, 2859; WO 97/47602 oder WO 920655 bzw.
J. Med. Chem. 1987, 30, 2111 und 2208.

Die Verbindungen der Formel (IV) sind entweder bekannt oder können nach bekannten Verfahren hergestellt werden, wie z.B. beschrieben in A.R. Katrizky, C.W. Rees (ed.) "Comprehensive Heterocyclic Chemistry", Pergamon Press, oder "The Chemistry of Heterocyclic Compounds" J. Wiley & Sons Inc. NY und der dort zitierten Literatur oder in S. Kubota et al. Chem. *Pharm*. *Bull* 1975, 23, 955 oder Vosilevskii et al. Izv. Akad. Nauk. SSSR Ser. Khim 1975, 23, 955.

Die nachfolgenden Beispiele dienen zur Erläuterung der Erfindung ohen sie zu begrenzen.

### Beispiel 1

### 4'-Acetyl-biphenyl-4-carbonsäure-N-(4-(4-(2-methoxyphenyl)-piperazin-1-yl)but-1-yl)amid

### Beispiel 2

### Thienyl-2-carbonsäure-N-(4-(4-(2,3-dichlorphenyl)piperazin-1-yl)but-1-yl)amid

### Beispiel 3

### 3-(4-(4-(2-t-Butyl-6-trifluormethyl)pyrimidin-4-yl)piperazin-1-yl)but-2-yl-mercapto)-4-methyl-5-methylamino-1,2,4 (4H)triazol

### Beispiel 4

### 3-(4-(4-(2-t-Butyl-6-trifluormethyl)pyrimidin-4-yl)piperazin-1-yl)2-methylen-propy-1-yl-mercapto)5-methylamino-1,2,4(4H)-triazol

### Beispiel 5

### 5-Amino-3-(4-(4-(2-t-Butyl-6-trifluormethyl)pyrimidin-4-yl)-homopiperazin-1-yl)2-methyl-but-2-en-1-yl-mercapto)-4-methyl-1,2,4(4H)triazol

### Beispiel 6

### 3-(4-(4-(2-t-Butyl-6-trifluormethyl)pyrimidin-4-yl)piperazin-1-yl)prop-1-yl-mercapto)-4-methyl-5-methylamino-1,2,4 (4H)triazol

### Beispiel 7

### 5-Amino-3-(4-(4-(2-t-Butyl-6-trifluormethyl)pyrimidin-4-yl)-piperazin-1-yl)2-methyl-but-2-en-1-yl-mercapto)-4-methyl-1,2,4(4H)triazol

### Beispiel 8

### 5-Amino-3-(4-(4-(2-t-Butyl-6-n-propyl)pyrimidin-4-yl)piperazin-1-yl)2-methyl-prop-1-en-1-yl-mercapto)-4-methyl-1,2,4(4H)triazol

### Beispiel 9

### 5-Amino-3-(4-(4-(2-t-Butyl-6-trifluormethyl)pyrimidin-4-yl)-piperazin-1-yl)2-methyl-but-2-yl-mercapto)-4-methyl-1,2,4(4H)triazol

### Beispiel 10

### 2-(3-(4-(2-t-Butyl-6-trifluormethyl)pyrimidin-4-yl)piperazin-1-yl)propoxy-pyrimidin-4-ol

### Beispiel 11

### 2-(3-(4-(2-t-Butyl-6-trifluormethyl)pyrimidin-4-yl)piperazin-1-yl)propylmercaptopyrimidin-4-ol-fumarat

### Beispiel 12

### 2-Naphtosäure-N-(4-(4-(2-t-Butyl-6-trifluormethyl)pyrimidin-4-yl)homopiperazin-1-yl)but-1-yl-amid

### Beispiel 13

### 5-Amino-3-(4-(4-(2-t-butyl-6-n-propyl)pyrimidin-4-yl)piperazin-1-yl)-2-methylen-prop-1-yl-mercapto)-4-methyl-1,2,4 (4H)triazol

### Beispiel 14

### 3-(4-(4-(2-t-butyl-6-trifluormethyl)pyrimidin-4-yl)piperazin-1-yl)prop-1-yl-mercapto-5-(2,5-dimethylfur-3-yl)4-methyl-1,2,4-(4H)triazol

### Beispiel 15

### (4-(4-(2-t-butyl-6-trifluormethyl)pyrimidin-4-yl)homopiperazin-1-yl)prop-1-yl-mercapto-4-methyl-5-(4-methyl-pyrazol-5-yl)1,2,4-(4H)triazol

### Beispiel 16

### 4-Methyl-5-phenyl-1,2,4 (4H)-triazol-3-carbonsaeure-N-(4-(4-(2-t-butyl-6-trifluormethyl)pyrimidin-4-yl)homopiperazin-1-yl)but-1-yl-amid

### Beispiel 17

### 5-Amino-2-(8-(4-(3-cyanophenyl)piperazin-1-yl)octyl-1-ylmercapto)1,3,4-thiadiazol

### Beispiel 18

### 2-(3-(5-(3-(Trifluormethylphenyl)-1,5-diazozin-1-yl)-propylmercapto)-pyrimidin-4-ol

### Beispiel 19

### 3-(4-(4-(2-t-Butyl-6-trifluormethyl)pyrimidin-4-yl)piperazin-1-yl)but-1-yl-pyrimidin-4-ol

### Beispiel 20

### 4-Methoxybenzoesäure-N-(4-(4-(2-t-butyl-6-trifluormethyl)-pyrimidin-4-yl)homopiperazin-1-yl)but-1-yl-amid

### Beispiel 21

### 1-Benzthiophen-2-carbonsaeure-N-(4-(4-(2-t-butyl-6-trifluormethyl)pyrimidin-4-yl)piperazin-1-yl)but-1-yl-amid

### Beispiel 22

### 5-Methoxybenzofuran-2-carbonsäure-N-(4-(4-(2-t-butyl-6-trifluormethyl)pyrimidin-4-yl)homo-piperazin-1-yl)but-1-yl-amid

### Beispiel 23

### (E)-N-({2-[(7-cyano-3,4-dihydro-2(1H)-isochinolinyl)methyl]cyclopropyl}methyl)-3-(1H-indol-5-yl)-2-propenamid

### Beispiel 24

### 2-(4-{[(E)-3-(1H-indol-5-yl)-2-propenoyl]amino}butyl)-1,2,3,4-tetrahydro-7-isochinolinyl trifluoromethanesulfonate

Überraschenderweise kann durch die Verwendung solcher Dopamin-D₃-Rezeptorliganden zur Herstellung von Arzneimitteln zur Bekämpfung von Nierenfunktionsstörungen eine gezielte Verbesserung von pathophysiologischen Störungen der Filtration erreicht werden.

Die Wirkung der Dopamin-D₃-Antagonisten wurde in einem tierexperimentellen Modell der diabetischen Nephropathie untersucht. Ratten, bei denen durch Gabe von Streptozotozin ein Diabetes mellitus ausgelöst wurde, entwickelten innerhalb von vierzehn Tagen eine ausgeprägte glomuläre Hyperfiltration. Wenn Ratten, bei denen auf diese Weise ein Diabetes mellitus ausgelöst worden war, subchronisch mit einem Dopamin-D₃-Rezeptor-Antagonisten behandelt wurden, trat die diabetische Hyperfiltration nicht auf.

Die D₃-Rezeptorliganden können auch in Kombination mit anderen Wirkstoffen zur Herstellung von Arzneimitteln zur Behandlung der genannten Krankheitsbilder eingesetzt werden. Insbesondere eignen sich für eine solche Kombination ACE-Inhibitoren wie beispielsweise Trandolapril und AT₁-Antagonisten wie zum Beispiel Losartan. Weiterhin kann auch eine Kombination mit Calciumantagonisten oder mit β-Blockern erfolgen.

### Methoden

Männlichen Sprague-Dawley-Ratten mit einem Körpergewicht von 180 bis 200 g wurden 60 mg/kg Streptozotozin, aufgelöst in Zitratpuffer (21 mg Zitronensäure pro 1 ml aqua bidest.), intraperitoneal injiziert. Ein Diabetes mellitus galt als erfolgreich ausgelöst, wenn nach 24 Stunden im venösen Blut eine abendliche Glucosekonzentration von mindestens 180 mg/dl festgestellt wurde. Die Blutglucose-Werte der diabetischen Tiere lagen zwischen 350 und 450 mg/dl. Eine Insulinsubstitution war somit nicht notwendig. Die Versuchstiere nahmen ca. 5 g pro Tag an Körpergewicht zu. Nicht-diabetischen Zeitkontrolltieren (CON) wurde das gleiche Volumen an Zitratpuffer intraperitoneal injiziert. Sie wiesen Blutglukosewerte zwischen 90 und 1120 mg/dl auf.

Unmittelbar nach erfolgter Feststellung des Diabetes mellitus wurden die Tiere in normalen Käfigen bei Standardfutter (Altromin 1320) und Leitungswasser ad libitum untergebracht. Durch unterschiedliche medikamentöse Behandlung (Verabreichung der Substanzen über das Trinkwasser) wurden die folgenden vier Versuchsgruppen gebildet:
CON (1) nicht-diabetische Tiere ohne zusätzliche Behandlung (Zeitkontrolle)
DM-VHC (2) diabetische Tiere ohne zusätzliche Behandlung (diabetische Kontrolle)
DM-SUL (3) diabetische Tiere mit S-Sulpirid-Behandlung (25 mg/kg/d)
DM-D3 (4) diabetische Tiere mit D₃-A-Behandlung (D₃-Al:
   D₃-Rezeptorantagonist gemäß Beispiel (7) (5 mg/kg/d);
   D₃-A2: D₃-Rezeptorantagonist gemäß Beispiel (11)

Täglich wurde das aufgenommene Flüssigkeitsvolumen registriert. Jeden dritten Tag wurden das Körpergewicht sowie der Blutglucose-Spiegel der Tiere bestimmt. Nach 12 Tagen wurden die Versuchstiere für 24 Stunden zur Urinsammlung in Stoffwechselkäfigen gehalten. Die Bestimmung der Proteinkonzentration im Urin erfolgte nach der von Lowry beschriebenen Standardmethode. Hierzu wurde nach Inkubation der Proben mit 1M NaOH eine Farbreaktion mit Folinreagenz (Phenol-Ciocalteus) induziert, deren Intensität photometrisch quantifiziert wurde. Die Bestimmung der Albuminkonzentration im Urin erfolgte durch einen für Albumin spezifischen Radio-Immun-Assay (RIA). Eingesetzt wurde ein Kit der Firma Biermann (KHAD 2: Albumin-RIA), der einen polyklonalen Antikörper gegen humanes Serumalbumin (Ziege) und als Tracer ¹²⁵ J-markiertes humanes Serumalbumin verwendet. Nach 14 bis 16 Tagen wurden die Tiere mit Thiopental (80 mg/kg) narkotisiert und Polyethylen-Katheter in die Vena jugularis und die Arteria carotis sowie in die Harnblase eingelegt. Bestimmt wurde mittels der renalen ³H-Inulin-Clearance die glomeruläre Filtrationsrate (GFR). Weiterhin wurden während des Versuches fortlaufend der mittlere arterielle Blutdruck (MAP) und die Herzfrequenz (HF) registriert. Am Ende der Clearance-Experimente wurden die Nieren entnommen und einer histopathologischen Untersuchung unterzogen.

### Ergebnisse

In der 24-stündigen Urinsammlung von wachen Tieren zeigte sich bei den unbehandelten diabetischen Tieren gegenüber den Zeitkontrollen eine deutliche Proteinurie (133 vs. 46 mg/d) und eine noch deutlichere Albuminurie (118 vs. 23 µg/d) als Ausdruck der Schädigung der glomerulären Filtrationsbarriere. Bei den mit Sulpirid behandelten diabetischen Tieren war diese pathologische Albuminurie nur leicht abgeschwächt (101 µg/d) und die Proteinurie sogar noch gesteigert (178 mg/d). Im Gegensatz dazu war bei den mit D₃-A gemäß Beispiel 7 behandelten Tieren die Proteinurie vermindert (113 mg/d) und die Albuminurie mit nur 18 µg/d vollständig unterdrückt.

Die Selektivität für Sulpirid und D₃-A1 ist wie in der WO 96/02520 zu Beispiel 42 beschrieben.

In der Clearance-Untersuchung wiesen die narkotisierten unbehandelten diabetischen Tiere (2) eine gegenüber den Zeitkontrollen (1) deutlich gesteigerte glomeruläre Filtrationsrate (1.10 vs. 0,83 ml/min) als Zeichen der diabetischen glomerulären Hyperfiltration auf. Sowohl bei Sulpirid- als auch bei D₃-Abehandelten diabetischen Tieren ( (3) bzw. (4) ) war diese diabetische Hyperfiltration unterdrückt, die Tiere wiesen eine nahezu normale GFR auf (0,71 bzw. 0,83 ml/min).

Die histopathologische Untersuchung zeigte bei den nicht behandelten diabetischen Tieren (2) im Vergleich zu gesunden Zeitkontrollen (1) neben insgesamt hypertrophisch vergrößerten Nieren eine signifikante Vergrößerung der Glomerula (ausgedrückt durch den Mittelwert von je 20 mikroskopisch ausgemessenen Flächen). Diese glomeruläre Hypertrophie war bei den diabetischen Tieren mit Sulpirid-Behandlung (3) ebenfalls zu beobachten, jedoch nicht bei den diabetischen Tieren, die mit D₃-A1 behandelt worden waren (4).

Das normale Naßgewicht der Nieren von nicht diabetischen adulten Ratten betrug 0,9 ± 0,1 g pro 100 g Körpergewicht. Bei diabetischen Ratten (DM-VHC) betrug das Nieren-Naßgewicht 1,1 + 0,04 g pro 100 g Körpergewicht, was eine beginnende Hypertrophie anzeigt. Behandlung mit D₃-A2 normalisierte das Nieren-Naßgewicht auf 0,96 + 0,03 g pro 100 g Körpergewicht.

Zusammengefaßt zeigen diese an einem in-vivo-Modell des Diabetes mellitus erhobenen Daten, daß die subchronische Behandlung mit pharmakologischen Hemmstoffen von Dopamin-D₂-Rezeptoren (Sulpirid) und von Dopamin-D₃-Rezeptoren (D₃-A) zu einer signifikanten Verminderung der renalen hämodynamischen Veränderungen im Rahmen der diabetischen Nephropathie führt.

Subchronische Behandlung mit D₃-A2 (30 mg/kg Körpergewicht pro Tag) vermindert deutlich die glomeruläre Hyperfiltration und die Diabetes-induzierte renale Hyperperfusion. Ebenso wurde die beginnende renale Hypertrophie deutlich reduziert.

Der Vergleich der Behandlungsgruppen zeigte zudem, daß die bei diabetischer Nephropathie auftretenden strukturellen intrarenalen Veränderungen durch die Dopamin-D₃-Rezeptor-Antagonisten D₃-A1 und D₃-A2, nicht aber durch den Dopamin-D₂-Rezeptor-Antagonisten Sulpirid aufgehoben wurden. Funktionell äußerte sich dieser Unterschied auch in der durch den Dopamin-D₃-Rezeptor-Antagonisten D₃-A, nicht aber durch den Dopamin-D₂-Rezeptor-Antagonisten Sulpirid deutlich verringerten pathologischen Protein- und Albuminausscheidung, die als ein wichtiger Marker der zunehmenden Funktionsverschlechterung der Niere gilt. Insgesamt geben die Ergebnisse dieser Untersuchung klare Hinweise darauf, daß die Verabreichung von Dopamin-D₃-Rezeptor-Antagonisten wie z.B. D₃-A den Verlauf der diabetischen Nephropathie günstig beeinflussen kann.

## Patentansprüche

1. Verwendung von Dopamin-D₃-Rezeptor-Antagonisten zur Herstellung von Arzneimitteln zur Behandlung von Nierenfunktionsstörungen.

2. Verwendung nach Anspruch 1 zur Herstellung von Arzneimitteln zur Behandlung von Nierenfunktionsstörungen mit glomerulärer Hyperfiltration.

3. Verwendung nach Anspruch 1 zur Herstellung von Arzneimitteln zur Behandlung von durch Diabetes mellitus hervorgerufenen Nierenfunktionsstörungen.

4. Verwendung nach Anspruch 1 zur Herstellung von Arzneimitteln zur Behandlung von durch nichtrenale Hypertonie verursachten Nierenfunktionsstörungen.

5. Verwendung nach Anspruch 1 zur Herstellung von Arzneimitteln zur Behandlung von durch Glomerulonephritis verursachten Nierenfunktionsstörungen.

6. Verwendung nach Anspruch 1 zur Herstellung von Arzneimitteln zur Behandlung von durch aufsteigende Harnwegsinfekte hervorgerufenen Nierenfunktionsstörungen.

7. Verwendung nach Anspruch 1 zur Herstellung von Arzneimittteln zur Behandlung von durch Sichelzellanämie hervorgerufenen Nierenfunktionsstörungen.

8. Verwendung nach Anspruch 1 zur Herstellung von Arzneimitteln zur Behandlung von durch kompensatorische Hypertrophie nach unilateraler Nephrektomie hervorgerufenen Nierenfunktionsstörungen.

9. Verwendung nach Anspruch 1 zur Herstellung von Arzneimitteln zur Behandlung von durch mesangiale Dysfunktion hervorgerufenen Nierenfunktionsstörungen.

10. Verwendung von Dopamin-D₃-Rezeptor-Antagonisten zur Herstellung von Arzneimitteln zur Behandlung von diabetischer Nephropathie.

11. Verwendung nach einem der Ansprüche 1 bis 10, wobei der Rezeptor-Antagonist 5-Amino-3-(4-(4-(2-t-Butyl-6-trifluormethyl)pyrimidin-4-yl-)-piperazin-1-yl)2-methyl-but-2-en-1-ylmercapto)-4-methyl-1,2,4(4H)triazol oder 2-(3-(4-(2-t-Butyl-6-trifluormethyl)pyrimidin-4-yl-)-piperazin-1-yl)propylmercaptopyrimidin-4-ol-fumarat ist.

## Claims

1. Use of dopamine D₃ receptor antagonists for the production of drugs for the treatment of renal function disorders.

2. The use as claimed in claim 1 for the production of drugs for the treatment of renal function disorders with glomerular hyperfiltration.

3. The use as claimed in claim 1 for the production of drugs for the treatment of renal function disorders caused by diabetes mellitis.

4. The use as claimed in claim 1 for the production of drugs for the treatment of renal function disorders caused by nonrenal hypertension.

5. The use as claimed in claim 1 for the production of drugs for the treatment of renal function disorders caused by glomerulonephritis.

6. The use as claimed in claim 1 for the production of drugs for the treatment of renal function disorders caused by ascending urinary tracts infections.

7. The use as claimed in claim 1 for the production of drugs for the treatment of renal function disorders caused by sickle cell anemia.

8. The use as claimed in claim 1 for the production of drugs for the treatment of renal function disorders caused by compensatory hypertrophy after unilateral nephrectomy.

9. The use as claimed in claim 1 for the production of drugs for the treatment of renal function disorders caused by mesangial dysfunction.

10. The use of dopamine D₃ receptor antagonists for the production of drugs for the treatment of diabetic nephropathy.

11. The use as claimed in any of claims 1 to 10, where the receptor antagonist is
5-amino-3-(4-(4-(2-t-butyl-6-txifluoromethyl)pyrimidin-4-yl)-piperazin-1-yl)-2-methyl-but-2-en-1-yl-mercapto)-4-methyl-1,2,4-(4H)-triazole
or 2-(3-(4-(2-t-butyl-6-trifluoromethyl)pyrimidin-4-yl)piperazin-1-yl)propylmercaptopyrimidin-4-ol fumarate.

## Revendications

1. Utilisation d'antagonistes de récepteur de dopamine D₃ pour la préparation de médicaments pour le traitement de troubles de la fonction rénale.

2. Utilisation selon la revendication 1 pour la préparation de médicaments pour le traitement de troubles de la fonction rénale avec hyperfiltration glomérulaire.

3. Utilisation selon la revendication 1 pour la préparation de médicaments pour le traitement de troubles de la fonction rénale provoqués par le diabète sucré.

4. Utilisation selon la revendication 1 pour la préparation de médicaments pour le traitement de troubles de la fonction rénale provoqués par une hypertonie non rénale.

5. Utilisation selon la revendication 1 pour la préparation de médicaments pour le traitement de troubles de la fonction rénale provoqués par la glomérulonéphrite.

6. Utilisation selon la revendication 1 pour la préparation de médicaments pour le traitement de troubles de la fonction rénale provoqués par des infections ascendantes des voies urinaires.

7. Utilisation selon la revendication 1 pour la préparation de médicaments pour le traitement de troubles de la fonction rénale provoqués par une anémie à cellules falciformes.

8. Utilisation selon la revendication 1 pour la préparation de médicaments pour le traitement de troubles de la fonction rénale provoqués par une hypertrophie compensatrice après une néphrectomie unilatérale.

9. Utilisation selon la revendication 1 pour la préparation de médicaments pour le traitement de troubles de la fonction rénale provoqués par un dysfonctionnement mésangial.

10. Utilisation d'antagonistes de récepteur de Dopamine D₃ pour la préparation de médicaments pour le traitement de la néphropathie diabétique.

11. Utilisation selon l'une des revendications 1 à 10, où l'antagoniste de récepteur est le 5-amino-3-(4-(4-(2-t-butyl-6-trifluorométhyl)-pyrimidin-4-yl-)-pipérazin-1-yl)-2-méthyl-but-2-én-1-yl-mercapto)-4-méthyl-1,2,4(4H)triazole ou le fumarate de 2-(3-(4-(2-t-butyl-6-trifluorofométhyt)pyrimidin-4-yl-)-pipérazin-1-yl)propylmercaptopyrimidin-4-ol.
